(19) **Europäisches Patentamt**
**European Patent Office**
**Office européen des brevets**

(11) **EP 2 306 822 B1**

(12) **EUROPEAN PATENT SPECIFICATION**

(45) Date of publication and mention of the grant of the patent:
**26.10.2016 Bulletin 2016/43**

(51) Int Cl.:
*A01N 37/34* (2006.01)  *A01N 35/08* (2006.01)
*A01P 1/00* (2006.01)  *A61K 8/40* (2006.01)
*A61K 8/42* (2006.01)

(21) Application number: **09790327.2**

(22) Date of filing: **13.07.2009**

(86) International application number:
**PCT/US2009/050372**

(87) International publication number:
**WO 2010/009033 (21.01.2010 Gazette 2010/03)**

(54) **BIOCIDAL COMPOSITION AND METHOD**

BIOZIDZUSAMMENSETZUNG UND VERFAHREN

COMPOSITION BIOCIDE ET METHODE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **15.07.2008 US 80828 P**

(43) Date of publication of application:
**13.04.2011 Bulletin 2011/15**

(73) Proprietor: **Dow Global Technologies LLC Midland, MI 48674 (US)**

(72) Inventors:
• **RAYMOND, Jon, B.**
**Buffalo Grove**
**IL 60089 (US)**
• **ANNIS, Ioana**
**Mundelein**
**IL 60060 (US)**
• **ADNETT, Judith**
**Cleveland TS18 4DT (GB)**

(74) Representative: **Beck Greener**
**Fulwood House**
**12 Fulwood Place**
**London WC1V 6HR (GB)**

(56) References cited:
**US-A- 4 732 913**

• **EXNER J H ET AL: "RATES AND PRODUCTS OF DECOMPOSITION OF 2,2-DIBROMO-3-NITRILOPROPIONAMIDE" JOURNAL OF AGRICULTURAL AND FOOD CHEMISTRY, AMERICAN CHEMICAL SOCIETY, US LNKD- DOI:10.1021/JF60189A012, vol. 21, no. 5, 1 September 1973 (1973-09-01), pages 838-842, XP002053943 ISSN: 0021-8561**
• **LEGIN G. YA.: "2-Bromo-2-nitro-1,3-propanediol (Bronopol) and its Derivatives: Synthesis, Properties and Application (A Review)" PHARMACEUTICAL CHEMISTRY JOURNAL, vol. 30, no. 4, 1996, pages 273-284, XP002590400**
• **CHERVENAK MARY C. ; KONST GERALD B. ; SCHWINGEL WILLIAM R.: "Nontraditional use of the biocide DBNPA in coatings manufacture", JCT COATINGSTECH, vol. 2, no. 13, 1 February 2005 (2005-02-01), pages 38-42, XP009152238, US ISSN: 1547-0083**
• **"Standard Test Method for Resistance of Emulsion Paints in the Container to Attack by Microorganisms" In: "Standard Test Method for Resistance of Emulsion Paints in the Container to Attack by Microorganisms", 1 June 2006 (2006-06-01), ASTM International, XP055147301, pages 1-4,**
• **Jenny Lunenburg-Duindam ET AL: "In-can preservation of emulsion paints", European Coatings Journal, no. 3, 1 March 2000 (2000-03-01), pages 1-8, XP055147308,**

EP 2 306 822 B1

**(Cont. next page)**

• ANONYMOUS: 'Product Information Liquid Preservatives For Treatment Of Industrial Process Systems And Product Preservation Applications - BIOBAN BP-10/30' DOW, [Online] 01 October 2003, pages 1 - 8, XP055147290 Retrieved from the Internet: <URL:http://msdssearch.dow.com/PublishedLiteratureDOWCOM/dh_0048/0901b803800487a2.pdf? filepath=biocides/pdfs/noreg/253-01298.pdf& fromPage=GetDoc> [retrieved on 2014-10-17]

## Description

### Field of the Invention

The invention relates to a method for rapid decontamination and long term preservation of alkaline aqueous media. The method uses a blend of 2,2-dibromo-3-nitrilopropionamide and 2-bromo-2-nitro-1,3-propanediol.

### Background of the Invention

Industrial hygiene plays an important role in preventing bacterial contamination and bio-deterioration of finished products. The term industrial hygiene encompasses good housekeeping practices, microbial monitoring of materials and process equipment, and modes to decontaminate raw materials, process water, recycled material, finished products and process equipment.

For use in industrial hygiene applications, a biocidal composition should, at a minimum, exhibit rapid decontamination efficacy in order to be broadly applicable. For example, during periods of high production in paint manufacturing processes, the constant and rapid recycling of wash water demands that a biocide eliminate microorganisms in a relatively short time frame (minutes to hours). Furthermore, leftover raw material heels (e.g., latex, thickeners, mineral slurries, and surfactants) in bulk storage tanks are commonly spoiled and can subsequently serve as a contamination source upon addition and contact with fresh raw materials. Thus, application of a biocide with rapid decontamination activity is beneficial for the sanitization of raw material heels immediately prior to replenishing storage tanks. In addition to rapid decontamination, it is also important for the biocide to remain functional over extended periods to protect against further inoculation with microorganisms. And, since aqueous materials that require microbial decontamination are often alkaline, the biocide composition should be capable of providing this prolonged preservation under such alkaline conditions.

Currently, the products used for decontamination fall into two categories: 1) fast acting, and quickly degradable biocides such as 2,2-dibromo-3-nitrilopropionamide (DBNPA) based products, where most often the decontamination occurs in less than one hour; and 2) slower acting and longer lasting, such as CMIT/MIT-based products (mixtures of 5-chloro-2-methyl-3-isothiazolone and 2-methyl-3-isothiazolone), where decontamination takes place in approximately 24 hours and the antimicrobial is stable in the system for over a week. In this second family of offerings, are blends of CMIT/MIT and 2-bromo-2-nitro-1,3-propanediol (BNPD). These blends benefit from the quicker antimicrobial action of BNPD and longer term preservation of CMIT/MIT. However, CMIT/MIT is a known sensitizer.

DBNPA is a desirable biocide because it is a fast acting, low cost material that exhibits efficacy against a broad spectrum of microorganisms. It is known, however, that DBNPA undergoes rapid hydrolytic degradation in basic solution. For instance, Exner et al., J. Agr. Food. Chem., 1973, 21(5), 838-842 ("Exner") teaches that DBNPA is "stable under acidic conditions, but the rate of disappearance increases by a factor of about 450 in going from pH 6, essentially neutral, to pH 8.9, slightly basic. At pH 11.3, the half life ($t_{1/2}$) for the disappearance of DBNPA is 25 sec, essentially instantaneous." (See Exner, page 839, left column). At pH 8, the half life of DBNPA is reported to be 2 hours (at 25 °C). See Exner, page 839, Table 1.

BNPD exhibits high activity, efficacy against a broad spectrum of microorganisms, and low mammalian toxicity at in-use levels, and is therefore also a highly desirable biocide. BNPD, however, is known to undergo some decomposition at alkaline pH. As a consequence, suppliers of BNPD generally recommend a use pH range that is non, or only slightly, basic. For example, Clariant's product literature for Nipaguard® BNPD recommends a pH range of 3.0 to 7.0. The Dow Chemical Company's product literature for Bioban™ Bronopol (a BNPD product) teaches that the greatest long-term chemical stability with the material is achieved at pH levels between about 4 to 8.

Blends of DBNPA and BNPD have been reported in U.S. Patent 4,732,913 to be useful in various applications, such as cooling water and pulp and paper manufacture. These applications, however, require only a fast acting biocide that rapidly decontaminates the system. Long term preservation, particularly where the system is reinnoculated with microorganisms following the initial decontamination, is not generally a concern.

There is a need in the market-place for a non-sensitizing biocide that will combine rapid microorganism decontamination (minimum 4 $\log_{10}$ reduction of microorganism concentration in 1 hour) with extended protection from future microorganism insult for at least one week. In addition, the biocide should provide these characteristics under alkaline conditions.

### BRIEF SUMMARY OF THE INVENTION

In one aspect, the invention provides a method for reducing the microorganism concentration in an alkaline aqueous medium by at least 4 $\log_{10}$ within 1 hour and protecting against future microorganism insult for at least one week. The method comprises including in the aqueous medium an effective amount of a biocidal mixture comprising 2,2-dibromo-3-nitrilopropionamide (DBNPA) and 2-bromo-2-nitro-1,3-propanediol (BNPD).

### DETAILED DESCRIPTION OF THE INVENTION

As noted above, the invention provides methods for reducing the microorganism concentration in an alkaline aqueous medium, where the medium requires both rapid decontamination and long term preservation.

As used herein, rapid decontamination means a minimum 4 $\log_{10}$ reduction of microorganism concentration within 1 hour. As used herein, long term preservation means that the aqueous medium is resistant to at least one microorganism insult that occurs anytime up to one week after incorporation of the biocide blend in the medium.

In order to provide both rapid decontamination and long term preservation of microorganism (e.g., bacteria, yeast, fungi and algae) growth in alkaline aqueous media, the invention provides for the incorporation in such media of a biocidal mixture comprising 2,2-dibromo-3-nitrilopropionamide (DBNPA) and 2-bromo-2-nitro-1,3-propanediol (BNPD). The inventors have discovered that this biocidal blend surprisingly provides both rapid decontamination and long term preservation for up to one to two weeks in alkaline matrices. Thus, alkaline aqueous media contaminated with microorganisms are found by the inventors to be quickly decontaminated by the biocide blend, and the treated medium further resists contamination by a second microorganism inoculation administered, for example, three or seven days later. By "alkaline" is meant that the pH of the aqueous media is above 7. Preferably, the pH is 7.5 or above, more preferably 8 or above. In further embodiments, the pH is 8.5 or above, or 9 or above.

The inventors' discovery is surprising because, according to the teachings of the prior art, both DBNPA and BNPD rapidly decompose at alkaline pH. These biocides, therefore, would not be expected to exhibit extended efficacy at such pH. As noted above, DBNPA by itself has a half-life of only about 2 hours at pH 8 and the recommended use range for BNPD is pH 3-8. The inventors' have discovered that a DBNPA/BNPD blend surprisingly does in fact show strong preservative power at time points previously believed to be unachievable by these alkaline-labile molecules.

Rapid decontamination and long term preservation at alkaline pH is important in a number of fields, including industrial hygiene and various other applications, such as the decontamination and preservation of raw materials, finished products and process/wash water used in the manufacture of finished products, as well as in equipment and tank clean-up. Examples of suitable industries include paints and coatings, latexes, mineral slurries, adhesives, detergents, cleaners, cleaning wipes, car care products, and in the raw materials for these products. Other suitable industries include oil and gas exploration, fracture fluid preservation, leather tanning, and personal care products. Cooling tower water, pulp and paper applications, and lakes and lagoons, are excluded from the invention as these are industries where long-term preservation is not generally needed.

Preferably, the weight ratios of DBNPA:BNPD used in the invention is between about between 100:1 to 1:100, more preferably 16:1 to 1:16, and even more preferably 5:1 to 1:5. In further preferred embodiments, the weight ratio (DBNPA:BNPD) is between about 5:1 and 2:1, particularly preferably the ratio is about 3:1.

Preferably the two biocides are preblended at the preferred ratio above and dosed into the aqueous medium as a single product. The biocides may, however, also be added to the aqueous medium individually, although this is less preferred. The use concentration for the blend can be readily determined by a person of ordinary skill in the art. Preferably the use concentration is between about 100 ppm and 4000 ppm, more preferably between about 125 and 2000 ppm, by weight based on the total weight of the aqueous medium. In certain instances, either rapid decontamination or long-term preservation may be desired (versus both). In these cases the amount of biocide needed can be readily adjusted accordingly.

Preferably, the biocides are formulated together as a blend in water and glycol or other suitable solvent. For this preferred embodiment, the total actives concentration for the blend is preferably between about 5% and about 90%, more preferably between about 10% and about 30%, by weight.

Other biocides may be incorporated in the aqueous medium. However, it is preferred that if CMIT/MIT is present, its concentration is less than 15 ppm, more preferably less than 5 ppm, based on the total weight of the aqueous medium. Even more preferably, the aqueous medium is free of CMIT/MIT. This is because CMIT/MIT is a known skin sensitizer.

As noted above, the DBNPA/BNPD blend is useful in alkaline aqueous media requiring both rapid decontamination and long term preservation. The blends are preferably used to decontaminate raw materials, finished products, process/wash water, and equipment used in various industries, including the following: paints and coating, latex, mineral slurries, adhesives, detergents, cleaners, cleaning wipes, and car care products.

The following examples are illustrative of the invention but are not intended to limit its scope.

EXAMPLES

The antimicrobial profiles of DBNPA, BNPD, and a combination of DBNPA-BNPD (3:1 w/w) are tested in a commercial water-based latex (UCAR Latex 626 available from The Dow Chemical Company), and a generic mineral slurry sample. Commercial latexes vary in pH values from slightly acidic to basic. For the purpose of this study, a latex with a basic pH (8.7) is selected, as it is known that both BNPD and DBNPA have limited chemical stability at alkaline pH. Mineral slurries vary in pH, but most often they are alkaline. For the purpose of this study, a titanium dioxide slurry with pH of 8.35 was selected.

Both the latex and the mineral slurry are free of contamination prior to testing. At time zero, the test matrices are inoculated with a pool of microorganisms (listed below) to a final concentration of $5 \times 10^6$ CFU/ml. Aliquots of the contaminated samples are weighed into a series of sterile containers followed by addition of the appropriate volume of biocide required to achieve the desired concentration. A control sample, lacking biocide, is included in each evaluation. Following biocide addition, samples are thoroughly mixed and aliquots are removed and streaked to tryptic soy agar plates, at various time points, for the enumeration of surviving microorganisms. All agar plates are incubated at 30°C for 72 hours prior to assessment of viable bacteria. In all the ensuing results, a minimum of 4 $\log_{10}$ reduction in microorganism concentration, as compared to the control sample, is considered significant efficacy.

*Microorganisms:* Twenty-four hour tryptic soy broth cultures are combined in equal parts for formulation inoculation at a final concentration of 5x10⁶ CFU/ml. Organisms utilized: *Pseudomonas aeruginosa* (ATCC#15442), *Pseudomonas aeruginosa* (ATCC#10145), *Staphylococcus aureus* (ATCC#6538), *Burkholderia cepacia* (ATCC#25416), *Pseudomonas fluorescens B* (environmental isolate), *Pseudomonas oleovorans* (environmental isolate), *Enterococcus sulfureus* (environmental isolate). To assess the rapid decontamination efficacy of the biocides, aliquots are removed at 60 minutes following the initial inoculation, and streaked on tryptic soy agar plates for the enumeration of surviving microorganisms. The samples are stored at ambient conditions for three or seven days, respectively, at which point a second inoculation is administered, to test the long term preservation efficacy of the biocides. Aliquots are removed at 4 h and/or 24 h or 48 h following the second inoculation, and streaked on tryptic soy agar plates, for the enumeration of surviving microorganisms.

*Synergy:* The synergy indexes reported here are measured and calculated using Formula 1. In this approach, a synergy index (SI) of 1 indicates additivity. If the synergy index is less than 1, synergy has occurred, while a synergy index greater than 1 indicates antagonism.

$$\text{Synergy Index} = C_A/C_a + C_B/C_b \qquad (1)$$

where:

$C_a$ = concentration of antimicrobial A, alone, producing a predetermined end-point
$C_b$ = concentration of antimicrobial B, alone, producing a predetermined end-point
$C_A$ and $C_B$ = the concentrations of antimicrobials A and B, respectively, together in a mixture, that produce a predetermined end-point

**Efficacy results for UCAR Latex 626.**

As expected, DBNPA alone, at 140 ppm active concentration, is effective in decontaminating the latex, after 1 h from the initial inoculum. BNPD, even at the highest tested active concentration (600 ppm), did not achieve 4 $\log_{10}$ reduction in bacterial concentration in 1 hour. A combination of 120 ppm active DBNPA and 35 ppm active BNPD effectively decontaminated the sample, and a synergy index of less than 0.92 was achieved at this time point. The true value of the combination, and very unexpected result was the behavior after the second inoculation at the three days timepoint. Four hours after this second inoculation, the maximum tested concentrations of individual actives did not achieve decontamination, while a combination of 177.5 ppm DBNPA and 59.2 ppm BNPD was effective (SI < 0.54). After 24 h from this second inoculation, BNPD (95.7 ppm active) was found effective. However the combination was effective at very low concentration of the two actives (SI = 0.29). Even more surprising, when the second inoculation was performed seven days from the time of the initial inoculation, relatively small concentrations of the two actives in combination achieved decontamination in 24 hours (80.9 ppm DBNPA and 27 ppm BNPD). DBNPA by itself did not achieve decontamination even at the highest tested concentration, while 95.7 ppm BNPD were required when used alone. A synergy index of 0.48 was calculated based on this data. This is truly unexpected because conventional wisdom would predict that the two actives would be deactivated at this later time-point.

Similar results are observed in mineral slurry. For these experiments, aliquots are removed after 30 min from the initial inoculation and assessed for bacterial content. Both DBNPA (at 23.4 ppm) and the DBNPA and BNPD (3:1 active wt ratio) combination ( 17.6 ppm DBNPA and 5.9 ppm BNPD) are found effective, while BNPD is not effective at the highest tested concentration of 600 ppm active at this very early timepoint. The synergy index for the combination is 0.76. Again, unexpectedly, the combination effectively protected the mineral slurry from a second inoculation administered seven days later. Thus, in aliquots analyzed 48 hours after this second inoculation, 400 ppm DBNPA or 35.1 ppm BNPD, when used alone, were effective at obtaining 4 $\log_{10}$ reduction in microorganisms. By comparison, a combination of 26.4 ppm DBNPA and 8.8 ppm BNPD achieved the desired 4 $\log_{10}$ reduction in microorganisms. Significant synergy (synergy index 0.32)) was observed at this later time-point, which supports the strength of the DBNPA-BNPD combination.

*Comparative Example*. In both matrices, CMIT/MIT by itself or in combination with BNPD (sold as Acticide LA) failed to achieve rapid decontamination as defined here. The two products were effective as long term preservatives (and in some instances exhibited synergy for long term preservation, but could not deliver rapid decontamination and prolonged preservation together. It is important to note, that 15 ppm CMIT concentration is the skin sensitization limit imposed by R43 in Europe (see Annex I of Directive 67/548/EEC).

## Claims

1. A method for reducing the microorganism concentration in an alkaline aqueous medium by at least 4 $\log_{10}$ within 1 hour and protecting against future microorganism insult for at least one week, the method comprising including in the alkaline aqueous medium an effective amount of a biocidal mixture comprising 2,2-dibromo-3-nitrilopropionamide (DBNPA) and 2-bromo-2-nitro-1,3-propanediol (BNPD).

2. A method according to claim 1 wherein the weight ratio of 2,2-dibromo-3-nitrilopropionamide to 2-bromo-2-nitro-1,3-propanediol is between 100:1 and 1:100.

3. A method according to claims 1-2 wherein the 2,2-dibromo-3-nitrilopropionamide and the 2-bromo-2-nitro-1,3-propanediol are included in the aqueous medium at a total initial concentration of between 100 ppm and 4000 ppm based on the total weight of the aqueous medium.

4. A method according to claims 1-3 wherein the alkaline aqueous medium has a pH of 7.5 or above.

5. A method according to claims 1-4 wherein the alkaline aqueous medium is raw materials, finished products and process/wash water used in the manufacture of finished products, as well as in equipment and tank clean-up in paints and coatings, latexes, mineral slurries, adhesives, detergents, cleaners, cleaning wipes, car care products, and in the raw materials for these products, oil and gas exploration, fracture fluid preservation, leather tanning, and personal care products.

6. A method according to claims 1-5 wherein the alkaline aqueous medium is treated only once over a one week period with the biocidal mixture.

7. A method according to claims 1-6 wherein the aqueous medium contains less than 15 ppm of CMIT/MIT.

8. A method according to claims 1-7 wherein the aqueous medium is inoculated with one or more microorganism insults following incorporation of the biocidal blend therein.

## Patentansprüche

1. Ein Verfahren zum Reduzieren der Konzentration von Mikroorganismen in einem wässrigen alkalischen Medium um mindestens 4 $\log_{10}$ innerhalb 1 Stunde und Schützen vor künftigem Befall durch Mikroorganismen für mindestens eine Woche, wobei das Verfahren das Einschließen einer wirksamen Menge eines bioziden Gemischs, das 2,2-Dibrom-3-nitrilopropionamid (DBNPA) und 2-Brom-2-nitro-1,3-propandiol (BNPD) beinhaltet, in dem wässrigen alkalischen Medium beinhaltet.

2. Verfahren gemäß Anspruch 1, wobei das Gewichtsverhältnis von 2,2-Dibrom-3-nitrilopropionamid zu 2-Brom-2-nitro-1,3-propandiol zwischen 100 : 1 und 1 : 100 liegt.

3. Verfahren gemäß den Ansprüchen 1-2, wobei das 2,2-Dibrom-3-nitrilopropionamid und das 2-Brom-2-nitro-1,3-propandiol, bezogen auf das Gesamtgewicht des wässrigen Mediums, in dem wässrigen Medium in einer Gesamtausgangskonzentration von zwischen 100 ppm und 4000 ppm eingeschlossen sind.

4. Verfahren gemäß den Ansprüchen 1-3, wobei das wässrige alkalische Medium einen pH-Wert von 7,5 oder mehr aufweist.

5. Verfahren gemäß den Ansprüchen 1-4, wobei es sich bei dem wässrigen alkalischen Medium um Rohmaterialien, Fertigerzeugnisse und Betriebs-/Waschwasser, verwendet bei der Herstellung von Fertigerzeugnissen, sowie bei Reinigung von Ausrüstung und Tank, bei Farben und Beschichtungen, Latexen, Mineralschlämmen, Haftstoffen, Waschmitteln, Reinigungsmitteln, Reinigungstüchern, Produkten zur Autopflege und bei den Rohmaterialien für diese Produkte, Öl- und Gasaufschluss, Frac-Flüssigkeits-Konservierung, Ledergerbung und Produkte zur Körperpflege handelt.

6. Verfahren gemäß den Ansprüchen 1-5, wobei das wässrige alkalische Medium nur einmal im Verlauf eines einwöchigen Zeitraums mit dem bioziden Gemisch behandelt wird.

7. Verfahren gemäß den Ansprüchen 1-6, wobei das wässrige Medium weniger als 15 ppm CMIT/MIT enthält.

8. Verfahren gemäß den Ansprüchen 1-7, wobei das wässrige Medium mit einem oder mehreren Befällen durch Mikroorganismen, im Anschluss an die Aufnahme von biozider Mischung darin, okuliert wird.

## Revendications

1. Une méthode pour réduire la concentration en microorganismes dans un milieu aqueux alcalin d'au moins 4 $\log_{10}$ en 1 heure et pour protéger contre des attaques de micro-organismes futures pendant au moins une semaine, la méthode comprenant le fait d'inclure dans le milieu aqueux alcalin une quantité efficace d'un mélange de biocide comprenant du 2,2-dibromo-3-nitrilopropionamide (DBNPA) et du 2-bromo-2-nitro-1,3-propanediol (BNPD).

2. Une méthode selon la revendication 1 dans laquelle le rapport en poids du 2,2-dibromo-3-nitrilopropionamide au 2-bromo-2-nitro-1,3-propanediol est compris entre 100/1 et 1/100.

3. Une méthode selon les revendications 1 à 2 dans laquelle le 2,2-dibromo-3-nitrilopropionamide et le 2-bromo-2-nitro-1,3-propanediol sont inclus dans le milieu aqueux à une concentration initiale totale d'environ 100 ppm et 4 000 ppm rapporté au poids total du milieu aqueux.

4. Une méthode selon les revendications 1 à 3 dans laquelle le milieu aqueux alcalin présente un pH de 7,5 ou plus.

5. Une méthode selon les revendications 1 à 4 dans laquelle le milieu aqueux alcalin consiste en des matières premières, des produits finis et des eaux de traitement/lavage utilisées dans la fabrication de produits finis, ainsi que dans le nettoyage d'équipement et de réservoir dans les secteurs des peintures et des revêtements, des latex, des boues minérales, des adhésifs, des détergents, des agents nettoyants, des lingettes nettoyantes, des produits pour l'entretien de voiture, et dans les matières premières pour ces produits, dans les secteurs de l'exploration pétrolière et gazière, de la préservation de fluide de fracturation, du tannage du cuir, et des produits de soin personnel.

6. Une méthode selon les revendications 1 à 5 dans laquelle le milieu aqueux alcalin est traité, uniquement une fois sur une période d'une semaine, avec le mélange biocide.

7. Une méthode selon les revendications 1 à 6 dans laquelle le milieu aqueux contient moins de 15 ppm de CMIT/MIT.

8. Une méthode selon les revendications 1 à 7 dans laquelle le milieu aqueux est inoculé d'une ou de plusieurs attaques de micro-organismes à la suite de l'incorporation du mélange homogène biocide dans celui-ci.

**EP 2 306 822 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4732913 A

**Non-patent literature cited in the description**

- **EXNER et al.** *J. Agr. Food. Chem.,* 1973, vol. 21 (5), 838-842